(19) Europäisches Patentamt
European Patent Office
Office européen des brevets

(11) **EP 3 568 170 B1**

(12) **EUROPÄISCHE PATENTSCHRIFT**

(45) Veröffentlichungstag und Bekanntmachung des Hinweises auf die Patenterteilung:
**01.03.2023** Patentblatt **2023/09**

(21) Anmeldenummer: **18700986.5**

(22) Anmeldetag: **10.01.2018**

(51) Internationale Patentklassifikation (IPC):
***A61M 1/16*** *(2006.01)* ***B01D 65/10*** *(2006.01)*
***G01M 3/32*** *(2006.01)*

(52) Gemeinsame Patentklassifikation (CPC):
**A61M 1/1692; B01D 65/102; G01M 3/3236;**
A61M 2205/3331; A61M 2205/705; B01D 2311/14

(86) Internationale Anmeldenummer:
**PCT/EP2018/050597**

(87) Internationale Veröffentlichungsnummer:
**WO 2018/130592 (19.07.2018 Gazette 2018/29)**

(54) **EXTRAKORPORALE BLUTBEHANDLUNGSVORRICHTUNG UND VERFAHREN ZUR ÜBERWACHUNG DER INTEGRITÄT EINES DIALYSATORS EINER EXTRAKORPORALEN BLUTBEHANDLUNGSVORRICHTUNG**

EXTRACORPOREAL BLOOD TREATMENT DEVICE AND METHOD FOR MONITORING THE INTEGRITY OF A DIALYZER OF AN EXTRACORPOREAL BLOOD TREATMENT DEVICE

DISPOSITIF DE TRAITEMENT EXTRACORPOREL DU SANG ET PROCÉDÉ POUR SURVEILLER L'INTÉGRITÉ D'UN DIALYSEUR D'UN DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.01.2017 DE 102017000227**

(43) Veröffentlichungstag der Anmeldung:
**20.11.2019** Patentblatt **2019/47**

(73) Patentinhaber: **Fresenius Medical Care Deutschland GmbH**
**61352 Bad Homburg v.d.H (DE)**

(72) Erfinder:
• **HÄCKER, Jürgen**
**61267 Neu-Anspach (DE)**
• **GRONAU, Sören**
**65428 Rüsselsheim (DE)**

(74) Vertreter: **Oppermann, Frank**
**OANDO Oppermann & Oppermann**
**Wilhelminenstrasse 1a**
**65193 Wiesbaden (DE)**

(56) Entgegenhaltungen:
**EP-A1- 1 327 457 WO-A1-97/11770**

**Beschreibung**

Die Erfindung betrifft eine extrakorporale Blutbehandlungsvorrichtung zur Durchführung einer extrakorporalen Blutbehandlung, bei der Blut in einem extrakorporalen Blutkreislauf durch die Blutkammer eines Dialysators strömt, der durch eine semipermeable Membran in die Blutkammer und eine Dialysatkammer unterteilt ist. Darüber hinaus betrifft die Erfindung ein Verfahren zur Überwachung der Integrität eines Dialysators einer extrakorporalen Blutbehandlungsvorrichtung.

Bei der Dialyse durchströmt das zu behandelnde Blut in einem extrakorporalen Blutkreislauf die Blutkammer eines durch eine semipermeable Membran in die Blutkammer und eine Dialysatkammer unterteilten Dialysators, während in einem Fluidsystem Dialysat die Dialysatkammer des Dialysators durchströmt. Der extrakorporale Blutkreislauf weist eine Blutzuführleitung auf, die zu der Blutkammer führt, und eine Blutabführleitung auf, die von der Blutkammer abgeht, und das Fluidsystem weist eine zu der Dialysatkammer führende Dialysatzuführleitung und eine von der Dialysatkammer abgehende Dialysatabführleitung auf. Die Leitungen können Teil eines zur einmaligen Verwendungen bestimmten Schlauchsystems sein.

In dem Fluidsystem ist zum Fördern des Dialysats mindestens eine Pumpe vorgesehen. Das frische Dialysat wird in einer Dialysatquelle bereitgestellt und gebrauchtes Dialysat in einen Ablauf abgeführt. Darüber hinaus weisen die Blutbehandlungsvorrichtungen im Allgemeinen eine Bilanziereinheit zum Bilanzieren von frischem und gebrauchtem Dialysat und eine Ultrafiltrationseinrichtung mit einer Ultrafiltratpumpe zum Entziehen von Ultrafiltrat aus dem Fluidsystem auf. Das System für die Zufuhr und Abfuhr des Dialysats, das beispielsweise eine oder mehrere Schlauchleitungen, Pumpen oder Absperrorgane umfassen kann, wird auch als Hydrauliksystem bezeichnet.

Während der Dialysebehandlung ist die Druckdifferenz vom Blut zum Dialysat im Allgemeinen über die Länge der Membran positiv. Wenn die Membran des Dialysators beschädigt sein sollte bzw. Fehlstellen aufweisen sollte, besteht die Gefahr, dass während der Blutbehandlung Blut aus dem extrakorporalen Blutkreislauf in das Hydrauliksystem der Blutbehandlungsvorrichtung gelangt. In diesem Fall sprechen die bekannten Blutleckdetektoren an, die im Hydrauliksystem flussab des Dialysators vorgesehen sind. Wenn Blut im Hydrauliksystem detektiert werden sollte, muss die Blutbehandlung unterbrochen und der Dialysator ausgetauscht werden. Darüber hinaus sind aufwendige Desinfektionsmaßnahmen des Hydrauliksystems erforderlich.

Zur Überprüfung der Dichtigkeit eines Fluidsystems sind Druckhaltetests bekannt, bei denen im Fluidsystem ein Überdruck oder Unterdruck aufgebaut wird. Wenn in einer anschließenden Druckhaltetestphase der Druck in einem vorgegebenen Zeitintervall nicht unter einen bestimmten Grenzwert absinkt bzw. nicht über einen bestimmten Grenzwert ansteigt, kann darauf geschlossen werden, dass das Fluidsystem dicht ist.

Die WO 97/11770 A1 beschreibt extrakorporale Blutbehandlungsvorrichtung, die einen extrakorporalen Blutkreislauf, der die Blutkammer eines Dialysators einschließt, und ein Fluidsystem umfasst, das die Dialysatkammer des Dialysators einschließt. Die Blutbehandlungsvorrichtung verfügt über ein Pumpensystem zum Füllen des Fluidsystems mit einer Flüssigkeit und zum Aufbau einer Druckdifferenz zwischen der Blutkammer und der Dialysatkammer, und eine Druckmesseinrichtung zum Messen des Drucks in dem Fluidsystem für die Durchführung eines Integritätstests zur Überwachung der Integrität des Dialysators. In einem der Blutbehandlung vorausgehenden Vorbereitungsmodus werden sowohl die Blutkammer als auch die Dialysatkammer mit einer Spülflüssigkeit gefüllt, so dass die Membran des Dialysators sowohl auf der Blutseite als auch auf der Dialysatseite mit der Spülflüssigkeit benetzt ist. Für den Integritätstest wird eine Ultrafiltration durchgeführt und der Druck wird auf der Dialysatseite überwacht. Wenn durch die Ultrafiltration der Unterdruck auf der Dialysatseite nicht unter einen vorgegebenen Grenzwert absinkt, wird darauf geschlossen, dass die Membran des Dialysators nicht dicht ist.

Aus der EP 1 327 457 A1 ist eine Blutbehandlungsvorrichtung bekannt, die über eine Überwachungseinrichtung zur Detektion einer Leckage in dem Fluidsystem verfügt. Die Überwachungseinrichtung weist einen Drucksensor zum Messen des Drucks im Fluidsystem auf. Während der Blutbehandlung wird der Druck im Fluidsystem fortlaufend gemessen, aus einer möglichen Änderung des Drucks die Leckrate bestimmt, aus der Leckrate das Leckage-Volumen berechnet und das Leckage-Volumen mit einem vorgegebenen Grenzwert verglichen. Aus der EP 1 327 457 A1 ist auch ein Modell bekannt, das den statischen Druck im Flüssigkeitssystem unter Berücksichtigung der Compliance beschreibt.

Der Erfindung liegt die Aufgabe zugrunde, eine spätere Unterbrechung einer Blutbehandlung zu vermeiden, wenn für die Blutbehandlung ein defekter Dialysator verwendet werden sollte, dessen semipermeable Membran Fehlstellen aufweisen sollte.

Die Lösung dieser Aufgabe erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Vorrichtung und das erfindungsgemäße Verfahren beruhen auf der Erkenntnis, dass eine mit Flüssigkeit benetzte hydrophile Membran für Luft undurchlässig ist, solange deren Poren eine bestimmte Größe nicht überschreiten.

Die Menge der Luft, die pro Zeiteinheit durch eine Fehlstelle in der semipermeablen Membran hindurchtritt, lässt auf die Größe der Fehlstelle schließen. Der Zusammenhang zwischen Lochgröße und Volumenstrom wird näherungsweise durch das Hagen-Poiseuille Gesetz $dV/dt = \pi\, r^4/8\; \eta\; \Delta p/1$ beschrieben, wobei $\Delta p$ die

Druckdifferenz, η die dynamische Viskosität der Luft, r die Porengröße und 1 die Porenlänge der Membran ist.

Unter einer semipermeablen Membran wird nachfolgend jede Art von Membran verstanden, die beispielsweise in Plattendialysatoren und Hohlfaserdialysatoren oder Hohlkammerfiltern eingesetzt werden. Hohlkammerfilter weisen ein Bündel von Holfasern auf, das in einem Gehäuse angeordnet ist, wobei das Blut während der Behandlung im Allgemeinen durch die Hohlfasern fließt und das Dialysat im Gehäuse fließt. Die Bereiche innerhalb und außerhalb der Fasern sind mit Ausnahme der porösen Faserwände fluiddicht voneinander getrennt, so dass Flüssigkeit nur durch die Faserwände aus dem Inneren der Fasern in den Außenbereich gelangen kann. Die vorliegende Erfindung erfasst ebenfalls Dialysatoren, bei denen das Blut außerhalb der Fasern und das Dialysat innerhalb der Fasern fließt.

Die erfindungsgemäße Blutbehandlungsvorrichtung weist ein bei Betrieb die Dialysatkammer einschließendes Fluidsystem und ein Pumpensystem zum Füllen des die Dialysatkammer einschließenden Fluidsystems und zum Aufbau einer Druckdifferenz zwischen der Blutkammer und der Dialysatkammer auf, wobei das Pumpensystem mindestens eine Pumpe aufweist.

Für die Erfindung ist nicht wesentlich, wie die mindestens eine Pumpe zum Befüllen des Fluidsystems oder zum Aufbau der Druckdifferenz angesteuert wird. Zum Messen des Drucks in dem die Dialysatkammer einschließenden Fluidsystem und die Durchführung eines Integritätstests zur Überwachung der Integrität des Dialysators weist die Blutbehandlungsvorrichtung eine Druckmesseinrichtung auf. Eine Druckmesseinrichtung, die einen oder mehrere Drucksensoren aufweisen kann, ist in einer Blutbehandlungsvorrichtung im Allgemeinen vorhanden.

Das die Dialysatkammer einschließende Fluidsystem kann Teil eines größeren Fluidsystems, der Hydraulik, sein. Für die erfindungsgemäße Blutbehandlungsvorrichtung und das erfindungsgemäße Verfahren muss das die Dialysatkammer anschließende Fluidsystem lediglich die semipermeable Membran umfassen, da dessen Integrität bewertet werden soll, und es muss eine Druckmesseinrichtung vorgesehen sein, um den Druck in diesem Fluidsystem zu ermitteln. Ferner muss dieses Fluidsystem mit Flüssigkeit befüllbar sein, durch entsprechende Maßnahmen einerseits ein Teil der Flüssigkeit aus diesem Bereich entnehmbar sein und ein Druckgradient zwischen der Blutseite der semipermeablen Membran und der Dialysatseite der semipermeablen Membran erzeugbar sein.

Darüber hinaus weist die Blutbehandlungsvorrichtung eine mit dem Pumpensystem und der Druckmesseinrichtung zusammenwirkende Steuer- und Recheneinheit auf, die einen die Blutbehandlung vorbereitenden Vorbereitungsmodus vorsieht. Unter einem Behandlungsmodus wird der Betriebszustand der Blutbehandlungsvorrichtung verstanden, in dem die eigentliche Blutbehandlung erfolgt, d. h. dass Blut des Patienten durch die Blutkammer des Dialysators strömt.

Die Überwachung der Integrität des Dialysators erfolgt nicht während der Blutbehandlung, wenn der Patient an den extrakorporalen Blutkreislauf angeschlossen ist und Blut des Patienten durch die Blutkammer des Dialysators strömt, sondern vor der Blutbehandlung in dem Vorbereitungsmodus, der von der Steuer- und Recheneinheit vorgegeben wird. Wenn eine Fehlstelle in der Membran des Dialysators erkannt werden sollte, kann der Dialysator noch vor dem Anschließen des Patienten oder bevor das Blut die Blutkammer durchströmt gewechselt werden, so dass die Blutbehandlung später nicht unterbrochen zu werden braucht. Mit der Überprüfung der Integrität des Dialysators vor dem Beginn der Blutbehandlung wird die Sicherheit für den Patienten noch dadurch weiter erhöht, dass eine Blutbehandlung mit einem fehlerhaften Dialysator erst gar nicht begonnen werden kann.

Die Steuer- und Recheneinheit ist für die Durchführung des Vorbereitungsmodus derart konfiguriert, dass das die Dialysatkammer einschließende Fluidsystem mit einer Flüssigkeit befüllt wird, nach dem Befüllen des die Dialysatkammer einschließenden Fluidsystems mit einer Flüssigkeit der Integritätstest zur Überwachung der Integrität des Dialysators durchgeführt wird und nach der Durchführung des Integritätstests die Blutkammer mit einer Flüssigkeit befüllt wird. Folglich findet der Integritätstest zu einem Zeitpunkt statt, zu dem die Blutkammer noch nicht mit einer Flüssigkeit befüllt worden ist. Daher ist die semipermeable Membran des Dialysators vor der Durchführung des Integritätstests auf der Blutseite nicht mit Flüssigkeit benetzt worden. Mit anderen Worten, die Blutkammer ist weitestgehend trocken. Erst nach dem Integritätstest erfolgt die Befüllung der Blutkammer mit einer Flüssigkeit, beispielsweise mit einer Vorbereitungsflüssigkeit (Spülflüssigkeit), insbesondere Dialysat oder eine andere physiologische Flüssigkeit, um die Blutbehandlungsvorrichtung für die Durchführung der Blutbehandlung vorzubereiten. Nach dem Anschluss des Patienten an den extrakorporalen Blutkreislauf kann die Spülflüssigkeit dann von dem Blut des Patienten verdrängt werden.

Die Erfindung beruht auf der Erkenntnis dass sich eine Membran eines Dialysators für den Integritätstest anders verhält, wenn die Blutkammer noch nicht mit einer Flüssigkeit zuvor befüllt worden ist, d. h. die Membran auf der Blutseite nicht benetzt worden ist bzw. benetzt ist. Mit anderen Worten, eine Blutkammer mit der Membran, die einmal gefüllt wurde und anschließend wieder geleert wurde, verhält sich anders in dem Integritätstest als eine Membran einer zuvor nicht befüllten Membran. Die Membran ist bei einer Benetzung mit einer Flüssigkeit nur auf einer Seite oder auf beiden Seiten unterhalb eines bestimmten Grenzdrucks für Luft undurchlässig. Es hat sich gezeigt, dass sich bei einer Benetzung nur auf einer Seite die Testzeit des Integritätstests verkürzen lässt.

Für die Durchführung des Integritätstests ist die Steuer- und Recheneinheit in dem Vorbereitungsmodus derart konfiguriert, dass mit dem Pumpensystem zwischen der Blutkammer und der Dialysatkammer eine Druckdifferenz derart aufgebaut wird, dass der Druck in der Blutkammer größer als der Druck in der Dialysatkammer ist. Für den Integritätstest ist eine Druckdifferenz über die Membran des Dialysators entscheidend. Der Aufbau der Druckdifferenz kann dadurch erfolgen, dass auf der Blutseite ein Überdruck aufgebaut wird oder auf der Dialysatseite ein Unterdruck aufgebaut wird. Der Aufbau eines Überdrucks kann durch Zufuhr und der Aufbau eines Unterdrucks durch Abfuhr von Flüssigkeit aus der jeweiligen Kammer des Dialysators erfolgen.

Das Pumpensystem kann wenigstens eine Pumpe zum Füllen der Dialysatkammer und wenigstens eine zweite Pumpe aufweisen zum Erzeugen der Druckdifferenz. Dabei kann die Druckdifferenz beispielsweise durch eine Pumpe erfolgen, durch die Flüssigkeit aus der Dialysatkammer entzogen werden kann oder die Druckdifferenz kann durch eine Pumpe erfolgen, durch die Luft oder Flüssigkeit einem mit der Blutkammer fluidisch verbundenen Fluidsystem zugeführt werden kann, wodurch der Druck in der Blutkammer erhöht werden kann, ohne dass Flüssigkeit in die Blutkammer eintritt.

Bei einer bevorzugten Ausführungsform ist die Steuer- und Recheneinheit derart konfiguriert ist, dass für den Aufbau der Druckdifferenz zwischen der Blutkammer und der Dialysatkammer mit dem Pumpensystem dem die Dialysatkammer einschließenden Fluidsystem oder dem Teil des die Dialysatkammer einschließenden Fluidsystems Flüssigkeit entzogen wird, so dass in der Dialysatkammer ein Unterdruck gegenüber der Blutkammer aufgebaut wird. Zum Aufbau eines Überdrucks in der Blutkammer kann bei der alternativen Ausführungsform Luft oder Flüssigkeit dem nach außen abgeschlossenen extrakorporalen Blutkreislauf zugeführt werden. Dem Blutschlauchsystem kann beispielsweise Luft über eine am Entlüftungsport angeschlossene Luftzuführeinrichtung oder Substituat mittels einer Substituatpumpe zugeführt werden.

Zur Vorbereitung der Blutbehandlung wird das die Dialysatkammer des Dialysators einschließende Fluidsystem zunächst mit einer Flüssigkeit, beispielsweise Dialysat, befüllt. Während des Befüllens kann in der Dialysatkammer befindliche Luft über die Membran des Dialysators in das offene Schlauchsystem des extrakorporalen Blutkreislaufs abgeführt werden. Sobald sämtliche Poren der Membran auf der Dialysatseite mit Flüssigkeit benetzt sind, steigt der Druck in der Dialysatkammer stark an, wenn das Fluidsystem ein abgeschlossenes Volumen ist, da eine mit Flüssigkeit benetzte Membran, deren Poren eine bestimmte Größe nicht überschreiten, für Luft undurchlässig ist.

Nach dem Befüllen des die Dialysatkammer einschließenden Fluidsystem oder des Teils des Fluidsystems mit einer Flüssigkeit kann das Fluidsystem oder ein Teil des Fluidsystems mit der Flüssigkeit gespült werden. Die Spülung des Fluidsystems ist für die Überprüfung der Integrität des Dialysators aber nicht zwingend erforderlich. Durch das Spülen kann aber Luft aus der Dialysatkammer entfernt werden, die einen Einfluss auf die spätere Bestimmung der Compliance und den Integritätstest ausüben kann. Das Ausspülen der Luft erfolgt in diesem Fall beispielsweise in Richtung des Ablaufs des Dialysats aus der Dialysatkammer.

Für die Überprüfung der Integrität des Dialysators braucht nicht das gesamte Fluidsystem, das die Dialysatkammer des Dialysators einschließt, genutzt zu werden. Ausreichend ist, wenn der die Dialysatkammer einschließende Teil des Hydrauliksystems befüllt wird und in diesem Teil der Test erfolgt. Hierzu kann dieser Teil von dem übrigen Teil des Fluidsystems abgetrennt werden. Die Abtrennung der betreffenden Teile des Fluidsystems kann dadurch erfolgen, dass von der Steuer- und Recheneinheit im Fluidsystem vorgesehene Absperrorgane betätigt werden.

Nachdem die Membran während des Befüllvorgangs dialysatkammerseitig mit Flüssigkeit benetzt worden ist, wird bei der bevorzugten Ausführungsform dem die Dialysatkammer einschließenden Fluidsystem oder dem Teil des Fluidsystems Flüssigkeit entzogen, so dass sich in dem Fluidsystem oder dem Teil des Fluidsystems ein Unterdruck gegenüber der Blutkammer aufbaut, da eine intakte Membran für Luft undurchlässig ist. Es sei darauf hingewiesen, dass es sich hier um einen Druckhaltetest für die Membran handelt, so dass, wenn ein Unterdruck beschrieben wird, sich dieser auf das Verhältnis der Drücke zwischen Dialysatkammer und Blutkammer bezieht. Die absoluten Drücke im Hinblick auf den Atmosphärendruck sind unerheblich. Zur Bestimmung der Druckdifferenz zwischen Dialysatkammer und Blutkammer kann eine Druckmesseinrichtung für die Blutkammer vorgesehen sein. Solange sich jedoch der Druck auf der Blutseite nicht verändert, weil sie beispielsweise belüftet ist oder keine oder nur eine geringe Luftverschiebung von oder in die Blutseite erfolgt, können auch alleine die Druckänderungen auf der Dialysatseite herangezogen werden. Daher umfassen sämtliche vorliegenden Beschreibungen von Druckveränderungen und deren Messungen bzw. Ermittlungen auch den Fall, dass anstelle des Drucks auf der Dialysatseite alleine eine Druckdifferenz zwischen Dialysatseite und Blutseite Verwendung findet.

Die Steuer- und Recheneinheit ist derart konfiguriert, dass die Zunahme des mit der Druckmesseinrichtung gemessenen Drucks in einem vorgegebenen Zeitintervall ermittelt wird und aus der Zunahme des Drucks in einem vorgegebenen Zeitintervall und der Compliance des die Dialysatkammer einschließenden Fluidsystems eine Leckage-Rate des Fluidsystems ermittelt wird. Die Compliance des die Dialysatkammer einschließenden Fluidsystems kann eine vorgegebene (bekannte) Größe sein, die in einem Speicher der Steuer- und Recheneinheit gespeichert sein kann, oder kann eine Größe sein, die in dem Vorbereitungsmodus ermittelt wird.

Auf eine mangelnde Integrität des Dialysators wird dann auf der Grundlage der ermittelten Leckage-Rate geschlossen, bei der die Compliance des Fluidsystems Berücksichtigung findet. Diese Leckage-Rate sollte unterhalb eines Grenzwertes liegen, wenn der Dialysator intakt ist. Dabei wird davon ausgegangen, dass die Membran eines Dialysator, die dicht ist, d. h. deren Poren eine bestimmte Größe nicht überschreiten, für Luft undurchlässig ist, so dass sich beim Entziehen von Flüssigkeit ein Unterdruck aufbauen lässt.

In der Praxis hat sich gezeigt, dass die Compliance des Fluidsystems einen nicht zu vernachlässigenden Einfluss auf Druckänderungen ausübt. Daher liegt der Erfindung das Prinzip zugrunde, bei der Ermittlung der Leckage-Rate die Compliance des Fluidsystems oder des Teils des Fluidsystems, das die Dialysatkammer einschließt, zu berücksichtigen. Die Eigenschaften des Dialysators oder unterschiedlicher Schlauchsets oder Kassetten aber auch Verfahrenseigenschaften wie der Füllprozess können daher bei der Überwachung mitberücksichtigt werden. Unter Berücksichtigung der Compliance ist daher eine genaue Leckagemessung möglich. Da nur die Hydraulikseite des Dialysators mit Flüssigkeit befüllt wird, die Blutseite des Dialysators aber nicht mit Flüssigkeit befüllt ist, kann eine fehlerhafte Leckagemessung infolge eines Übertritts von Flüssigkeit durch die Membran des Dialysators verhindert werden. Darüber hinaus kann die Messzeit verkürzt werden. Je nach Ausgestaltung der Integritätsprüfung kann die Compliance des Fluidsystems gleichzeitig mit dem Entziehen von Flüssigkeit ermittelt werden.

Die Compliance kann auf der Grundlage einer Druckänderung infolge einer Volumenänderung bestimmt werden. Dem Fluidsystem wird hierzu ein vorgegebenes Volumen an Flüssigkeit entzogen oder zugeführt werden, wobei auf der Grundlage der Druckänderung in dem Fluidsystem die Compliance berechnet werden kann. Dabei nehmen die Druckänderungen mit abnehmender Compliance zu bzw. mit zunehmender Compliance ab, d.h. je größer die Nachgiebigkeit bzw. niedriger die Steifigkeit des Systems ist, desto geringer sind die Druckänderungen. Beispielsweise sind die Druckänderungen relativ gering, wenn sich die Wandungen der Schlauchleitungen oder der Kassette relativ stark verformen. Wenn zur Bestimmung der Compliance aus dem Fluidsystem Flüssigkeit abgeführt wird, braucht der Druck in dem System nicht erhöht zu werden, so dass ein bestimmter Grenzdruck nicht überschritten werden kann.

Eine Ausführungsform der erfindungsgemäßen Blutbehandlungsvorrichtung sieht vor, dass die Steuer- und Recheneinheit derart konfiguriert ist, dass zur Überwachung der Integrität des Dialysators in dem Vorbereitungsmodus die Compliance aus dem Volumen der Flüssigkeit, die in dem vorgegebenen Zeitintervall dem die Dialysatkammer einschließenden Fluidsystem oder dem Teil des Fluidsystems entzogen wird, und der Abnahme des mit der Druckmesseinrichtung gemessenen Drucks berechnet wird.

Da solche Systeme lange Zeit benötigen können bis sich die Druckwerte stabilisieren, kann vorgesehen sein, dass die Änderung des Drucks in einem vorgegebenen Zeitintervall erfasst wird und/oder dass zur Bestimmung der Compliance ein Druckwert herangezogen wird, bei dem eine Druckänderungsrate unterhalb eines Grenzwerts liegt.

Für den Integritätstest kann die ermittelte Leckage-Rate mit einem vorgegebenen Grenzwert für die Leckage-Rate verglichen werden und auf eine Undichtigkeit geschlossen und ein die mangelnde Integrität des Dialysators signalisierendes Steuersignal erzeugt werden, wenn in einem vorgegebenen Zeitintervall der vorgegebenen Grenzwert für die Leckage-Rate nicht unterschritten wird. Wenn der vorgegebene Grenzwert für die Leckage-Rate schon vor Ablauf des vorgegebenen Zeitintervall unterschritten wird, kann der Test abgebrochen und auf einen ordnungsgemäßen Dialysator geschlossen und ein die ausreichende Integrität des Dialysators signalisierendes Steuersignal erzeugt werden.

Mit anderen Worten, es wird die Leckage-Rate kontinuierlich oder zu wenigstens einem bestimmten Zeitpunkt oder zu mehreren vorbestimmten Zeitpunkten ermittelt. Liegt eine oder mehrere der ermittelten Leckage-Raten unterhalb eines vorgegeben Grenzwerts, wird das System als dicht erachtet und im Nachfolgenden kann eine Behandlung erfolgen. Eine gewisse Leckage-Rate kann deshalb hingenommen werden, weil auch bei integrer Membran ein Druckabbau auf anderem Wege erfolgen kann, beispielsweise durch undichte Anschlüsse oder ein sich über die Zeit oder bei Temperaturschwankungen veränderndes System.

In Abhängigkeit von dem Steuersignal kann eine Mitteilung an den Benutzer erfolgen, beispielsweise ein Alarm ausgelöst werden, und/oder eine Mitteilung auf einem Bildschirm angezeigt werden, und/oder ein geeigneter Eingriff in die Maschinensteuerung vorgenommen werden.

Der Entzug der Flüssigkeit aus dem Fluidsystem zum Aufbau des Unterdrucks erfolgt vorzugsweise mit der Ultrafiltratpumpe einer Ultrafiltrationseinrichtung, um dem Fluidsystem Flüssigkeit entziehen zu können. Eine Ultrafiltrationseinrichtung ist im Allgemeinen ein Teil des Pumpensystems der bekannten Blutbehandlungsvorrichtungen. Die Flüssigkeit kann aber auch mit einer anderen Pumpe im Fluidsystem abgepumpt werden. Dabei ist es dem Fachmann bekannt, dass die Ultrafiltrationseinheit in eine Bilanziereinheit integriert sein kann. Dies kann durch erreicht werden, dass die Ultrafiltrationspumpe zugleich ein Teil des Bilanzierpumpensystems ist und die Ultrafiltration lediglich durch unterschiedliche Pumpengeschwindigkeiten für die Zufuhr und die Abfuhr von Flüssigkeit erfolgt.

Im Folgenden wird die Erfindung unter Bezugnahme auf die Figuren im Einzelnen beschrieben.

Es zeigen:

Fig. 1 ein Ausführungsbeispiel einer erfindungsgemäßen Blutbehandlungsvorrichtung in vereinfachter schematischer Darstellung,

Fig. 2 den Druck im Fluidsystem als Funktion der Zeit für verschiedene Dialysatoren, und

Fig. 3 den Druck im Fluidsystem als Funktion der Zeit für den erfindungsgemäßen Integritätstest mit einer nur Befüllung des Dialysators nur auf der Dialysatsseite mit einer Flüssigkeit und für einen Integritätstest, bei dem auch die Blutseite des Dialysators mit einer Flüssigkeit befüllt war.

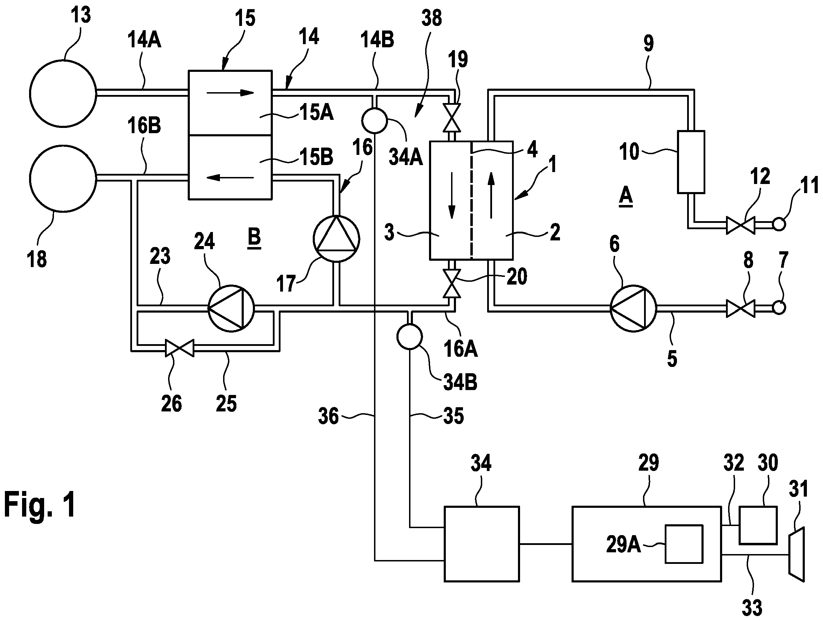

Fig. 1 zeigt die Komponenten einer erfindungsgemäßen Blutbehandlungsvorrichtung in stark vereinfachter schematischer Darstellung. Die Blutbehandlungsvorrichtung umfasst einen extrakorporalen Blutkreislauf A und ein Fluidsystem B. Der extrakorporale Blutkreislauf A schließt die Blutkammer 2 und das Fluidsystem B schließt die Dialysatkammer 3 eines Dialysators 1 ein. Blutkammer 2 und Dialysatkammer 3 des Dialysators 1 sind durch eine semipermeable Membran 4 getrennt sind. Eine Blutzuführleitung 5, in der eine Blutpumpe 6 vorgesehen ist, führt von einem arteriellen Patientenanschluss 7 zu dem Einlass der Blutkammer 2. Stromauf der Blutpumpe 6 kann sich an der Blutzuführleitung 5 ein arterielles Absperrorgan 8 befinden. Von dem Auslass der Blutkammer 2 führt eine Blutabführleitung 9, in die eine Flüssigkeitskammer (Tropfkammer) 10 geschaltet sein kann, zu einem venösen Patientenanschluss 11. Stromab der Flüssigkeitskammer 10 befindet sich an der Blutabführleitung 9 ein venöses Absperrorgan 12.

Der extrakorporale Blutkreislauf A und das Fluidsystem B oder Teile des extrakorporalen Blutkreislaufs oder des Fluidsystems B können als zur einmaligen Verwendung bestimmte Einmalartikel (Disposable) ausgebildet sein. Nachfolgend wird die Blutbehandlungsvorrichtung im aufgerüsteten Zustand beschrieben, in dem das oder die Disposables in die Blutbehandlungsvorrichtung eingelegt bzw. an dieser befestigt sind.

Das Fluidsystem B kann ferner eine Dialysatquelle 13 zur Bereitstellung von frischer Dialysierflüssigkeit aufweisen, die über einen ersten Abschnitt 14A einer Dialysatzuführleitung 14 mit dem Einlass einer Kammer 15A einer Bilanziereinheit 15 verbunden ist. Der zweite Abschnitt 14B der Dialysatzuführleitung verbindet den Auslass der einen Kammer 15A der Bilanziereinheit 15 mit dem Einlass der Dialysatkammer 3. Die Dialysierflüssigkeit kann als Spül- bzw. Vorbereitungsflüssigkeit verwendet werden.

Der Auslass der Dialysatkammer 3 ist über einen ersten Abschnitt 16A einer Dialysatabführleitung 16 mit dem Einlass der anderen Kammer 15B der Bilanziereinheit 15 verbunden. In dem ersten Abschnitt 16A der Dialysatabführleitung 16 befindet sich eine Dialysatpumpe 17. Der Auslass der anderen Kammer 15B der Bilanzereinheit 15 ist über den zweiten Abschnitt 16B der Dialysatabführleitung 16 mit einem Abfluss 18 verbunden. Die Bilanziereinheit 15 kann noch weitere Kammern aufweisen.

In der Dialysatzuführleitung 14 befindet sich stromauf der Dialysatkammer 3 ein Absperrorgan 19 und in der Dialysatabführleitung 16 stromab der Dialysatkammer 3 ein Absperrorgan 20. Die Absperrorgane 19, 20 können elektromagnetisch betätigbare Ventile sein. Von dem ersten Abschnitt 16A der Dialysatabführleitung 16 führt eine Ultrafiltratleitung 23, in der eine Ultrafiltratpumpe 24 vorgesehen ist, zu dem zweiten Abschnitt 16B der Dialysatabführleitung 16. Von einem Abschnitt der Ultrafiltratleitung 23 stromauf der Ultrafiltratpumpe 24 kann eine Bypassleitung 25, in der ein Bypass-Ventil 26 vorgesehen ist, zu einem Abschnitt der Ultrafiltratleitung 23 stromab der Ultrafiltratpumpe 24 führen. Die Blutpumpe 6, die Dialysatpumpe 17 und die Ultrafiltratpumpe 24 können okkludierende Pumpen sein. Wenn die Pumpen okkludierende Pumpen sind, können Absperrorgane für die Schaffung eines abgeschlossenen Volumens entfallen.

Die Blutzuführ- und -abführleitung 5, 9 des extrakorporalen Blutkreislaufs A sind bei dem vorliegenden Ausführungsbeispiel Bestandteil eines austauschbaren Blutschlauchsets und die Dialysatzuführ- und -abführleitung 14, 16 des Dialysierflüssigkeitssystems B sind fester Bestandteil des Hydrauliksystems der Blutbehandlungsvorrichtung. Das Blutschlauchset sowie der Dialysator 1 sind zur einmaligen Verwendung bestimmt und werden in nicht dargestellte Aufnahmen eingelegt. Anstelle eines Schlauchsets kann auch eine austauschbare Kassette vorgesehen sein, in der Kanäle für die Flüssigkeit, insbesondere die Zufuhr und Abfuhr von Blut, ausgebildet sind. Die Aufnahme für den Dialysator kann eine Halterung sein.

Die Blutbehandlungsvorrichtung verfügt über eine zentrale Steuer- und Recheneinheit 29 zur Steuerung sämtlicher Komponenten der Vorrichtung. Die zentrale Steuer- und Recheneinheit 29 kann beispielsweise einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte zur Steuerung der Blutbehandlungsvorrichtung auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich.

Die Steuer- und Recheneinheit 29 ist über nicht dargestellte Steuer- bzw. Datenleitungen mit den Pumpen 6, 17, 24, den Absperrorganen 8, 12, 19, 20 und dem Bypassventil 26 verbunden, so dass die Flussraten der Pumpen eingestellt und die Absperrorgane und das Bypassventil betätigt werden können. Die Pumpen 6, 17, 24, Absperrorgane 8, 12, 19, 20 und das Bypassventil 26 sind Teil eines Pumpensystems 38 der Blutbehandlungsvorrichtung.

Die Steuer- und Recheneinheit 29 weist einen Speicher 29A auf, in dem Messwerte und Parameter gespeichert werden können. Des Weiteren sind eine Anzeigeeinheit 30 und eine Alarmeinheit 31 vorgesehen, die über Datenleitungen 32, 33 mit der Steuer- und Recheneinheit 29 verbunden sind. Die Anzeigeeinheit 30 kann Anzeigeelemente, beispielsweise Signallampen, oder ein Display zur Anzeige verschiedener Symbole, aufweisen, die auf einen ordnungsgemäßen Dialysator oder eine mangelnde Integrität des Dialysators hinweisen. Die Alarmeinheit 31 kann einen Signalgeber für einen akustischen und/oder optischen und/oder taktilen Alarm aufweisen.

Der Druck im Fluidsystem B wird mit einer Druckmesseinrichtung 34 gemessen. Die Druckmesseinrichtung 34 kann über einen ersten Drucksensor 34A verfügen, der den Druck in dem zweiten Abschnitt 14B der Dialysatzuführleitung 14 misst, und/oder über einen zweiten Drucksensor 34B verfügen, der den Druck in dem ersten Abschnitt 16A der Dialysatabführleitung 16 misst. Die Steuer- und Recheneinheit 29 empfängt die Signale der Druckmesseinrichtung 32 über Datenleitungen 35, 36. Die gemessenen Druckwerte werden in dem Speicher 29A der Steuer- und Recheneinheit 29 für die Berechnung verschiedener Größen gespeichert.

Die bekannten Blutbehandlungsvorrichtungen sehen einen Behandlungsmodus und einen der Behandlung vorausgehenden Vorbereitungsmodus zur Vorbereitung der Behandlung vor. Während der eigentlichen Blutbehandlung ist der extrakorporale Blutkreislauf A, der die Blutzuführleitung 5, die Blutkammer 2 des Dialysators 1 und die Blutrückführleitung 9 umfasst, mit Blut befüllt, das von der Blutpumpe 6 gefördert wird. In dem der Blutbehandlung vorausgehenden Vorbereitungsmodus ist die Blutkammer nicht mit Blut befüllt.

Die Steuer- und Recheneinheit 29 ist derart konfiguriert, dass der Vorbereitungsmodus oder der Behandlungsmodus vorgegeben werden kann. Die Überwachung der Integrität der Membran 3 des Dialysators 1 erfolgt im Vorbereitungsmodus während eines Befüllungs- und Spülprozesses, der zur Vorbereitung der Blutbehandlung ohnehin durchgeführt wird. Für den Integritätstest steuert die Steuer- und Recheneinheit 29 die einzelnen Komponenten der Blutbehandlungsvorrichtung wie folgt an, um die nachfolgenden Verfahrensschritte durchzuführen.

Die Steuer- und Recheneinheit 29 öffnet das Absperrorgan 19 des Pumpensystems 38 in der Dialysatzuführleitung 14 stromauf der Dialysatkammer 3 und kann das Absperrorgan 20 in der Dialysatabführleitung 16 stromab der Dialysatkammer 3 schließen. Daraufhin wird Dialysat aus der Dialysatquelle über die Bilanziereinheit 15 der Dialysatkammer 3 zugeführt. Der Befülldruck der Dialysatkammer 3 wird mit dem Drucksensor 34A zum Messen des Drucks in der Dialysatzuführleitung 14 gemessen und mit der Steuer- und Recheneinheit 29 überwacht. Die in der Dialysatkammer 3 befindliche Luft kann dabei über die Membran 4 des Dialysators in die Blutkammer 2 und aus der Blutkammer über das offene Schlauchsystem des extrakorporalen Blutkreislaufs A entweichen. Sobald sämtliche Poren der Membran 4 benetzt sind, steigt der Druck in der Dialysatzuführleitung 14 stark an. Wenn der Befülldruck einen vorgegebenen Grenzwert, der in dem Speicher 29A gespeichert ist, überschreitet, öffnet die Steuer- und Recheneinheit 29 das Absperrorgan 20 in der Dialysatabführleitung 16, so dass die Dialysatkammer 3 kräftig durchspült wird, wobei das Dialysat in den Ablauf 18 strömt. Daraufhin sinkt der Druck in der Dialysatzuführleitung 14 wieder unter den Grenzwert.

Alternativ kann das Absperrorgan 20 auch geöffnet sein, und weitere Bereiche, je nach Stellung der vorhandenen Absperreinrichtungen, werden befüllt.

Das die Dialysatkammer 3 einschließende Fluidsystem B kann nunmehr mit Dialysat bei offener Klemme 20 weiter gespült werden, so dass das Hydrauliksystem weitergehend entlüftet ist. Am Ende des Spülvorgangs ist das Absperrorgan 19 in der Dialysatzuführleitung 14 wieder geschlossen. Die Membran 4 des Dialysators 1 ist auf der Hydraulikseite jetzt mit Dialysat benetzt, weshalb die Membran für Luft dicht ist. Auf der Blutseite ist die Membran aber nicht mit Flüssigkeit benetzt worden, da die Blutkammer noch nicht mit Blut befüllt worden ist. Bei einem Hohlkammerfilter sind die Lumen der Fasern der Membran nach wie vor leer. Dadurch kann der Integritätstest in relativ kurzer Zeit durchgeführt werden.

Nach dem Spülvorgang wird zumindest einem Teil des die Dialysatkammer 3 einschließenden Fluidsystems B Flüssigkeit entzogen, die bei dem vorliegenden Ausführungsbeispiel Dialysat als Spülflüssigkeit ist. Zum Entziehen von Flüssigkeit betreibt die Steuer- und Recheneinheit 29 die Ultrafiltratpumpe 24, wobei das Absperrorgan 19 in der Dialysatzuführleitung 14 geschlossen ist. Das Absperrorgan 26 in der Bypassleitung 25 ist ebenfalls geschlossen.

An dieser Stelle sei darauf hingewiesen, dass das Fluidsystem B erfindungsgemäß bei Betrieb lediglich die Dialysatkammer und eine Druckmesseinrichtung einschließen muss.

Weitere dargestellte Komponenten können vorhanden sein oder nicht bzw. können zu dem Fluidsystem B gehören oder nicht. Letztendlich kann jeder Bereich der Hydraulik zu dem erfindungsgemäßen Fluidsystem B gehören, solange ein mit Flüssigkeit befüllbarer und aus diesem Flüssigkeit entziehbarer Bereich gegeben ist, in dem der Druck gemessen werden kann und der auch die Dialysatkammer umfasst. Zudem sollte dieser Bereich auch geeignet sein, derart druckdicht absperrbar zu sein, dass die für den hier beschriebenen Drucktest erforderlichen Druckveränderungen ermittelbar sind.

Die Verwendung einer Ultrafiltratpumpe zum Entziehen der Flüssigkeit hat den Vorteil, dass sich mit dieser Pumpe ein bestimmtes Volumen an Flüssigkeit sehr genau entziehen lässt. Dies ist eine Voraussetzung für den bestimmungsgemäßen Gebrauch einer Ultrafiltratpumpe während der Blutbehandlung. Daher werden in Blutbehandlungsvorrichtungen als Ultrafiltratpumpen nur Pumpentypen eingesetzt, die bauartbedingt exakt definierbare Volumina dem System entziehen können. Bei der Ultrafiltratpumpe kann es sich um eine Membranpumpe handeln. Alternativ sind aber auch andere Pumpen einsetzbar. So kann auch eine Dialysatpumpe mit einem Flussmesser, beispielsweise mit einem Corioliskraftmesser, zum Abpumpen der Flüssigkeit eingesetzt werden.

Bei dem vorliegenden Ausführungsbeispiel wird in der Dialysatabführleitung 16 der Druck $p_{t1}$ vor einem oder jedem Pumpenhub und der Druck $p_{t2}$ nach einem oder jedem Pumpenhub gemessen. Für mindesten einen oder jeden Pumpentakt berechnet die Steuer- und Recheneinheit 29 aus dem bekannten Volumen dV eines Pumpenhubs und aus dem gemessenen Druck $p_{t1}$, $p_{t2}$ vor und nach dem jeweiligen Pumpenhub die Compliance C des den Dialysator 1 einschließenden Teils des Fluidsystem B. Die Berechnung der Compliancewerte, die in dem Speicher 29A gespeichert werden, erfolgt nach der folgenden Gleichung.

$$C = dV \, / \, (p_{t2} - p_{t1})$$

Für den Integritätstest ist es erforderlich, eine Druckdifferenz über die Membran zu erzeugen. In einer Ausführungsform wird beim Entziehen der Flüssigkeit mit der Ultrafiltratpumpe 24 in dem Fluidsystem B, das die Dialysatkammer 3 einschließt, ein Unterdruck aufgebaut, da die benetzte Membran 4 des Dialysators 1 für Luft weitgehend dicht ist, selbst wenn die Membran Fehlstellen aufweisen sollte, da das Volumen an Luft, die über die Fehlstellen in das Fluidsystem gelangen könnte, im Verhältnis zu dem Volumen der entzogenen Flüssigkeit gering ist.

Der Druck wird mit dem Drucksensor 34B in der Dialysatabführleitung 16 gemessen. Die Ultrafiltratpumpe 24 wird solange betrieben, bis sich ein vorgegebener Prüfdruck aufgebaut hat. Hierzu kann der momentan gemessene Druck mit dem vorgegebenen Prüfdruck laufend verglichen werden. Da die Compliance zumindest für den letzten Pumpenhub bestimmt wird, ist auch die Compliance bei dem Prüfdruck bekannt. Bei einer bevorzugten Ausführungsform wird der Compliancewert, der beim Prüfdruck ermittelt wird, der weiteren Auswertung zugrunde gelegt. Für diesen Fall ist es nicht erforderlich, die Compliancewerte für jeden Pumpenhub zu bestimmen. Es ist aber auch möglich, einen Mittelwert für die Compliance oder einen maximalen oder minimalen Compliancewert der weiteren Auswertung zugrunde zu legen.

Wenn sich der Prüfdruck auch nach einer vorgegebenen maximalen Fördermenge der Ultrafiltratpumpe 24 nicht aufgebaut hat, wird schon vorab darauf geschlossen, dass die Membran 4 des Dialysators 1 undicht ist. Auf eine Undichtigkeit kann auch geschlossen werden, wenn sich nach einem vorgegebenen Zeitintervall, in dem die Ultrafiltratpumpe läuft, der Prüfdruck nicht aufgebaut hat. Wenn dies der Fall ist, erzeugt die Steuer- und Recheneinheit 29 ein die mangelnde Integrität des Dialysators 1 signalisierendes Steuersignal, das die Anzeigeeinheit 30 und/oder die Alarmeinheit 31 empfängt. Auf der Anzeigeeinheit 30 kann dann angezeigt werden, dass die Integrität des Dialysators nicht gegeben ist, und/oder mit der Alarmeinheit 31 kann ein Alarm gegeben werden. Dann muss der defekte Dialysator gegen einen neuen Dialysator ausgetauscht werden.

Daraufhin erfolgt der eigentliche Integritätstest, mit dem sich in relativ kurzer Testzeit die Integrität des Dialysators sicher überprüfen lässt, wobei auch geringfügige Undichtigkeiten erkannt werden können.

Der Unterdruck nimmt während des Betriebs der Ultrafiltratpumpe 24 zu, mit anderen Worten die Druckdifferenz zur Blutkammer wird zunehmend größer. Wird der Prüfunterdruck für den Integritätstest erreicht, kann der Integritätstest beginnen. Dazu wird die zeitliche Veränderung des Druck in dem abgeschlossenen Fluidssystem B gemessen und die Druckanstiegsrate $\Delta p \, / \, t_A - t_B = p_{tA} - p_{tB} \, / \, t_A - t_B$ wird ermittelt, wobei $p_{tA}$ der Druck ist, der zum Zeitpunkt $t_A$ gemessen wird und $p_{tB}$ der zum späteren Zeitpunkt $t_B$ gemessene Druck ist, und $t_A - t_B$ das Zeitintervall zwischen Beginn der Messung bei $t_A$ und dem späteren Zeitpunkt $t_B$ ist

Aus der Druckanstiegsrate und der beispielsweise für den letzten Pumpenhub bestimmten Compliance C, d. h. der Compliance bei dem Prüfdruck, berechnet die Steuer- und Recheneinheit 29 die Leckage-Rate LR nach der folgenden Gleichung:

$$LR = C \, \Delta p \, / \, t_A - t_B$$

Indem diese Bestimmung von LR kontinuierlich oder zu einem oder mehr vorbestimmten Zeitpunkten erfolgen kann, wird eine gemittelte Leckage-Rate für das Zeitintervall $t_A$ bis $t_B$ berechnet.

Die berechnete Leckage-Rate LR wird mit einem vorgegebenen Grenzwert für die Leckage-Rate LR, der für einen intakten Dialysator angenommen wird, verglichen. Wenn die berechnete Leckage-Rate LR kleiner als der vorgegebene Grenzwert ist, wird darauf geschlossen, dass der Dialysator intakt ist. Dann kann die Steuer- und Recheneinheit 29 ein Steuersignal an die Anzeigeeinheit 30 senden, das die Integrität des Dialysators signalisiert, was auf der Anzeigeeinheit angezeigt wird. Die Steuer- und Recheneinheit 29 kann auch ein Steuersignal erzeugen, dass einen Eingriff in die Maschinensteuerung vornimmt. Beispielsweise kann nach Feststellung einer undichten Membran die Vorgabe des Behandlungsmodus verhindert bzw. nur bei Feststellung einer ausreichenden Integrität des Dialysators der Behandlungsmodus freigegeben werden.

Für den Integritätstest wird eine maximale Testzeit vorgegeben, in der zum Aufbau des Unterdrucks dem Fluidsystem Flüssigkeit entzogen wird. Wenn innerhalb der maximalen Testzeit die berechnete Leckage-Rate LR nicht kleiner als der vorgegebene Grenzwert ist, wird darauf geschlossen, das die Membran 4 undicht ist. In diesem Fall erzeugt die Steuer- und Recheneinheit 29 ein die mangelnde Integrität des Dialysators 1 signalisierendes Steuersignal, das die Anzeigeeinheit 30 und/oder die Alarmeinheit 31 empfängt, so dass auf der Anzeigeeinheit dann angezeigt wird, dass die Membran des Dialysators undicht ist, und mit der Alarmeinheit Alarm gegeben wird. Die Steuer- und Recheneinheit 29 kann auch ein Steuersignal erzeugen, das einen Eingriff in die Maschinensteuerung derart vornimmt, dass der Blutbehandlungsmodus nicht freigeben wird, so dass die Durchführung einer Behandlung mit einem defekten Dialysator nicht möglich ist.

Bei dem vorliegenden Ausführungsbeispiel wird die Ultrafiltratpumpe 24 taktweise betrieben. Es ist auch möglich, für jeden Takt (Pumpenhub) die Compliance C und die Leckage-Rate LR unter Berücksichtigung der Compliance C zu berechnen oder die Compliance und die Leckage-Rate für ein beliebiges vorgegebenes Zeitintervall zu bestimmen, dass mehrere Pumpenhübe umfassen kann.

Bei einer Leckage-Rate, die größer als null ist, kann der Prüfdruck im Fluidsystem leicht sinken. Dadurch kann das Messergebnis aber nur geringfügig verfälscht werden. Zur Vermeidung dieses Messfehlers kann die Steuer- und Recheneinheit 29 derart konfiguriert sein, dass die Ultrafiltratpumpe 24 derart angesteuert wird, dass der vorgegebene Prüfdruck, der in dem Teil des Fluidsystems, der die Dialysatkammer 3 einschließt, aufgebaut wurde, konstant gehalten wird. Hierzu wird eine Förderrate für die Ultrafiltratpumpe 24 eingestellt. Da sich der Prüfdruck entsprechend der Leckage-Rate abbaut, muss die Förderrate der Ultrafiltrationspumpe 24 gerade der Leckage-Rate entsprechen, um den Prüfdruck konstant zu halten. Bei dieser Ausführungsform weist die Steuervorrichtung eine druckbasierte Feedback Steuerung auf, um den Prüfdruck konstant zu halten. Bei dieser Ausführungsform muss die Blutbehandlungsvorrichtung bzw. das Verfahren also eingerichtet sein, ohne explizite Bestimmung der Compliance die Leckagerate zu bestimmen und daraus gemäß obiger Beschreibung den Integritätstest durchzuführen. Die Berücksichtigung der Compliance erfolgt dabei inherent in der Feedbacksteuerung, indem die Dynamik der Druckantwort des Systems bereits von der Compliance abhängt.

Bei einer weiteren Ausführungsform wird die Druckdifferenz durch Druckaufbau in der Blutkammer erzeugt. Dazu kann durch Zufuhr von Luft oder Flüssigkeit in den extrakorporalen Blutkreislauf (A) der Druck in der Blutkammer erhöht werden, ohne dass die Blutkammer mit Flüssigkeit befüllt wird. Ansonsten gelten die obigen Ausführungen zum Aufbau der Druckdifferenz durch Druckabbau in der Dialysatkammer analog.

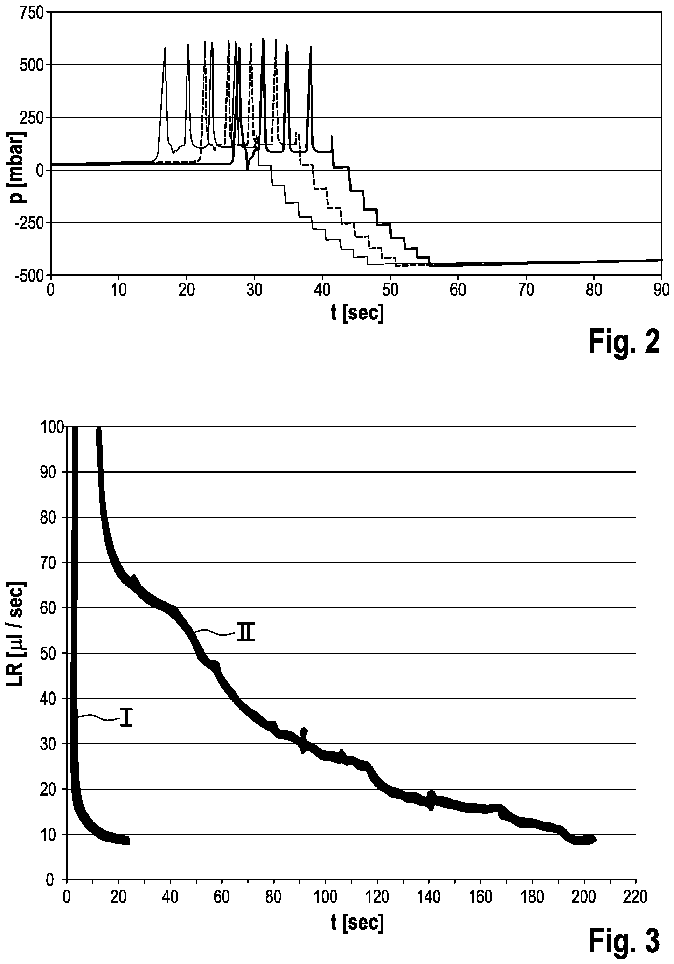

Fig. 2 zeigt den zeitlichen Verlauf des mit dem Drucksensor 34B in der Blutabführleitung 16 gemessenen Unterdrucks p, während die Ultrafiltratpumpe 24 dem Fluidsystem Flüssigkeit entzieht. In Fig. 2 ist der Unterdruck für drei verschiedene Dialysatortypen gezeigt. Es zeigt sich, dass der Unterdruck während des Entzugs von Flüssigkeit aus dem Fluidsystem zunimmt. Dabei erfolgt mit jedem Pumpenhub eine Zunahme des Unterdrucks stufenweise um einen bestimmten Betrag bis der Prüfdruck erreicht ist. Des Weiteren ist zu erkennen, dass nach dem letzten Ultrafiltrationspumphub ein leichter Anstieg erfolgt. Aus diesem Anstieg wird dann die Leckage-Rate LR bestimmt.

Fig. 3 zeigt den zeitlichen Verlauf der Leckage-Rate LR bei einer Messung mit einem Dialysator, dessen Dialysatkammer mit Flüssigkeit befüllt ist, wobei die Blutkammer einmal mit Flüssigkeit befüllt war (Graf II), im Vergleich zu einem Dialysator, dessen Dialysatkammer mit Flüssigkeit befüllt ist, wobei die Blutkammer nicht mit Flüssigkeit befüllt war (Graf I). Es zeigt sich, dass eine minimale Leckage-Rate LR bei einem Dialysator, dessen Blutkammer nicht mit Flüssigkeit befüllt war, schon nach ca. 20 Sekunden erreicht ist, während die minimale Leckage-Rate bei einem Dialysator, dessen Blutkammer mit Flüssigkeit befüllt war, erst nach ca. 200 Sekunden erreicht wird. Folglich kann die Messzeit für die Überprüfung der Integrität der Dialysatormembran mit dem erfindungsgemäßen Verfahren gegenüber einem konventionellen Verfahren deutlich verkürzt werden.

Nach dem Integritätstest wird der extrakorporale Blutkreislauf A mit einer Vorbereitungsflüssigkeit befüllt. Erst nach der Befüllung mit der Vorbereitungsflüssigkeit, beispielsweise Dialysat, wird die Membran des Dialysators auf auch der Blutseite mit Flüssigkeit benetzt. Zum Füllen der Blutkammer mit einer Vorflüssigkeit im Vorbereitungsmodus kann Flüssigkeit aus dem Hydrauliksystem B oder Flüssigkeit aus einem Behältnis dem extrakorporalen Blutkreislauf A zugeführt werden. Zum Spülen des extrakorporalen Kreislaufs A kann die Spülflüssigkeit über eine Verbindung dem Fluidsystem A zugeführt oder in ein separates Behältnis geleitet werden.

## Patentansprüche

1. Extrakorporale Blutbehandlungsvorrichtung zur Durchführung einer extrakorporalen Blutbehandlung, bei der Blut in einem extrakorporalen Blutkreislauf (A) durch die Blutkammer (2) eines Dialysators (1) strömt, der durch eine semipermeable Membran (4) in die Blutkammer (2) und eine Dialysatkammer (3) getrennt ist, wobei die Blutbehandlungsvorrichtung aufweist:

   ein wenigstens bei Betrieb die Dialysatkammer (3) einschließendes Fluidsystem (B), ein Pumpensystem (38) zum Füllen des die Dialysatkammer (3) einschließenden Fluidsystems (B) mit einer Flüssigkeit und zum Aufbau einer Druckdifferenz zwischen der Blutkammer (2) und der Dialysatkammer (3), wobei das Pumpensystem (38) mindestens eine Pumpe (6, 17, 24) aufweist,

eine Druckmesseinrichtung (34) zum Messen des Drucks in dem die Dialysatkammer (3) einschließenden Fluidsystem (B) für die Durchführung eines Integritätstests zur Überwachung der Integrität des Dialysators (1),

eine mit dem Pumpensystem (38) und der Druckmesseinrichtung (34) zusammenwirkende Steuer- und Recheneinheit (29), die einen die Blutbehandlung vorbereitenden Vorbereitungsmodus vorsieht,

**dadurch gekennzeichnet, dass** die Steuer- und Recheneinheit (29) für die Durchführung des Vorbereitungsmodus derart konfiguriert ist, dass das die Dialysatkammer (3) einschließende Fluidsystem (B) mit einer Flüssigkeit befüllt wird, nach dem Befüllen des die Dialysatkammer (3) einschließenden Fluidsystems (B) mit einer Flüssigkeit der Integritätstest zur Überwachung der Integrität des Dialysators (2) mit einer nur auf der Dialysatseite benetzten semipermeablen Membran (4) durchgeführt wird und nach der Durchführung des Integritätstests die Blutkammer (2) mit einer Flüssigkeit befüllt wird, wobei

die Steuer- und Recheneinheit (29) für die Durchführung des Integritätstests in dem Vorbereitungsmodus derart konfiguriert ist, dass zwischen der Blutkammer (2) und der Dialysatkammer (3) eine Druckdifferenz derart aufgebaut wird, dass der Druck in der Blutkammer größer als der Druck in der Dialysatkammer ist, die Zunahme des mit der Druckmesseinrichtung (34) gemessenen Drucks p in einem vorgegebenen Zeitintervall ermittelt wird, und

aus der Zunahme des Drucks in einem vorgegebenen Zeitintervall und der Compliance C des die Dialysatkammer (3) einschließenden Fluidsystems (B) eine Leckage-Rate LR ermittelt wird, und

auf eine mangelnde Integrität des Dialysators auf der Grundlage der ermittelten Leckage-Rate LR geschlossen wird.

2. Extrakorporale Blutbehandlungsvorrichtung nach Anspruch 1, **dadurch gekennzeichnet, dass** die Steuer- und Recheneinheit (29) derart konfiguriert ist, dass für den Aufbau der Druckdifferenz zwischen der Blutkammer (2) und der Dialysatkammer (3) mit dem Pumpensystem (38) dem die Dialysatkammer (3) einschließenden Fluidsystem (B) Flüssigkeit entzogen wird oder mit dem Pumpensystem (38) der Druck in der Blutkammer (2) durch Zufuhr von Luft oder Flüssigkeit in den extrakorporalen Blutkreislauf (A) erhöht wird.

3. Extrakorporale Blutbehandlungsvorrichtung nach Anspruch 1 oder 2, **dadurch gekennzeichnet, dass** die Steuer- und Recheneinheit (29) derart konfiguriert ist, dass die Leckage-Rate LR nach der folgenden Gleichung berechnet wird:

$$LR = C \, dp/dt$$

,wobei C die Compliance C des die Dialysatkammer einschließenden Fluidsystems (B) und dp/dt die Druckanstiegsrate ist.

4. Extrakorporale Blutbehandlungsvorrichtung nach Anspruch 1, 2 oder 3, **dadurch gekennzeichnet, dass** die Steuer- und Recheneinheit (29) derart konfiguriert ist, dass zur Überwachung der Integrität des Dialysators (1) in dem Vorbereitungsmodus die Compliance C aus einem Volumen der Flüssigkeit, die dem die Dialysatkammer (3) einschließenden Fluidsystem (B) entzogen wird, und der Abnahme des mit der Druckmesseinrichtung (34) gemessenen Drucks berechnet wird.

5. Extrakorporale Blutbehandlungsvorrichtung nach Anspruch 4, **dadurch gekennzeichnet, dass** die Steuer- und Recheneinheit (29) derart konfiguriert ist, dass die Compliance C nach der folgenden Gleichung berechnet wird:

$$C = dV/dp$$

,wobei dV das dem die Dialysatkammer einschließenden Fluidsystem (B) entzogene Volumen an Flüssigkeit und dp die Abnahme des Drucks vor Entzug und nach Entzug der Flüssigkeit ist.

6. Extrakorporale Blutbehandlungsvorrichtung nach einem der Ansprüche 1 bis 5, **dadurch gekennzeichnet, dass** die Steuer- und Recheneinheit (29) derart konfiguriert ist, dass die ermittelte Leckage-Rate LR mit einem vorgegebenen Grenzwert für die Leckage-Rate verglichen wird und auf eine mangelnde Integrität geschlossen wird, wenn in einem vorgegebenen Zeitintervall der vorgegebene Grenzwert für die Leckage-Rate nicht unterschritten wird.

7. Extrakorporale Blutbehandlungsvorrichtung nach einem der Ansprüche 2 bis 6, **dadurch gekennzeichnet, dass** das Pumpensystem (38) eine Ultrafiltratpumpe (24) aufweist, und dass die Steuer- und Recheneinheit (29) derart konfiguriert ist, dass die Ultrafiltratpumpe (24) zum Entziehen von Flüssigkeit aus dem die Dialysatkammer (3) einschließenden Fluidsystem (B) betrieben wird, so dass in dem die Dialysatkammer (3) einschließenden Fluidsystem (B) eine Druckabnahme erfolgt.

8. Extrakorporale Blutbehandlungsvorrichtung nach einem der Ansprüche 1 bis 7, **dadurch gekennzeichnet, dass** die Steuer- und Recheneinheit (29) derart konfiguriert ist, dass in dem Vorbereitungsmodus nach dem Befüllen des die Dialysatkammer (3) einschließenden Fluidsystems (B) mit einer Flüssigkeit das Fluidsystem gespült wird,

9. Verfahren zur Überwachung der Integrität eines Dialysators einer extrakorporalen Blutbehandlungsvorrichtung zur Durchführung einer extrakorporalen Blutbehandlung, bei der Blut in einem extrakorporalen Blutkreislauf durch die Blutkammer eines Dialysators strömt, der durch eine semipermeable Membran in die Blutkammer und eine Dialysatkammer getrennt ist,

   wobei die Blutbehandlungsvorrichtung einen die Blutbehandlung vorbereitenden Vorbereitungsmodus vorsieht, in dem das die Dialysatkammer einschließende Fluidsystem mit einer Flüssigkeit befüllt wird, nach dem Befüllen des die Dialysatkammer einschließenden Fluidsystems ein Integritätstest zur Überwachung der Integrität des Dialysators mit einer nur auf der Dialysatseite benetzten semipermeablen Membran durchgeführt wird und nach der Durchführung des Integritätstests die Blutkammer mit einer Flüssigkeit befüllt wird,
   wobei das Verfahren für den Integritätstest in dem Vorbereitungsmodus die folgenden Verfahrensschritte umfasst: mit einer nur auf der Dialysatseite benetzten semipermeablen Membran Aufbauen einer Druckdifferenz zwischen der Blutkammer und der Dialysatkammer derart, dass der Druck in der Blutkammer größer als der Druck in der Dialysatkammer ist,
   Ermitteln der Zunahme des Drucks p in dem die Dialysatkammer einschließenden Fluidsystem in einem vorgegebenen Zeitintervall, und
   Ermitteln aus der Zunahme des Drucks in einem vorgegebenen Zeitintervall und der Compliance C des die Dialysatkammer einschließenden Fluidsystems eine Leckage-Rate LR, und
   Erzeugen eines die mangelnde Integrität des Dialysators signalisierenden Steuersignals auf der Grundlage der ermittelten Leckage-Rate.

10. Verfahren zur Überwachung der Integrität eines Dialysators nach Anspruch 9,
    **dadurch gekennzeichnet, dass** zum Aufbauen einer Druckdifferenz zwischen der Blutkammer und der Dialysatkammer Flüssigkeit aus dem die Dialysatkammer einschließenden Fluidsystem entzogen wird oder mit dem Pumpensystem (38) der Druck in der Blutkammer (2) durch Zufuhr von Luft oder Flüssigkeit in den extrakorporalen Blutkreislauf (A) erhöht wird.

11. Verfahren zur Überwachung der Integrität eines Dialysators nach Anspruch 9 oder 10, **dadurch gekennzeichnet, dass** die Leckage-Rate LR nach der folgenden Gleichung berechnet wird:

$$LR = C \, dp/dt$$

,wobei C die Compliance C des die Dialysatkammer einschließenden Fluidsystems und dp/dt die Druckanstiegsrate ist.

12. Verfahren zur Überwachung der Integrität eines Dialysators nach Anspruch 9, 10 oder 11, **dadurch gekennzeichnet, dass** zur Überwachung der Integrität des Dialysators in dem Vorbereitungsmodus die Compliance C aus einem Volumen der Flüssigkeit, die dem die Dialysatkammer einschließenden Fluidsystem entzogen wird, und der Abnahme des gemessenen Drucks p berechnet wird.

13. Verfahren zur Überwachung der Integrität eines Dialysators nach Anspruch 12, **dadurch gekennzeichnet, dass** die Compliance C des Fluidsystems nach der folgenden Gleichung berechnet wird:

$$C = dV/dp$$

,wobei dV das dem die Dialysatkammer einschließenden Fluidsystem entzogene Volumen an Flüssigkeit und dp die Abnahme des Drucks vor Entzug und nach Entzug der Flüssigkeit ist.

14. Verfahren zur Überwachung der Integrität eines Dialysators nach einem der Ansprüche 9 bis 13, **dadurch gekennzeichnet, dass** die ermittelte Leckage-Rate LR mit einem vorgegebenen Grenzwert für die Leckage-Rate verglichen wird und ein die mangelnde Integrität des Dialysators signalisierendes Steuersignals erzeugt wird, wenn in einem vorgegebenen Zeitintervall der vorgegebenen Grenzwert für die Leckage-Rate nicht unterschritten wird.

15. Verfahren zur Überwachung der Integrität eines Dialysators nach einem der Ansprüche 9 bis 14, **dadurch gekennzeichnet, dass** nach dem Befüllen des die Dialysatkammer einschließenden Fluidsystems mit einer Flüssigkeit das Fluidsystem mit der Flüssigkeit gespült wird.

**Claims**

1. Extracorporeal blood treatment device for carrying out an extracorporeal blood treatment, in which blood flows in an extracorporeal blood circuit (A) through the blood chamber (2) of a dialyser (1) which is separated by a semi-permeable membrane (4) into the blood chamber (2) and a dialysate chamber (3), the blood treatment device comprising:

   a fluid system (B) including the dialysate chamber (3) at least during operation,
   a pump system (38) for filling the fluid system (B) including the dialysate chamber (3) with a liquid and for establishing a pressure difference between the blood chamber (2) and the dialysate chamber (3), the pump system (6, 17, 24) having at least one pump (38),
   a pressure measuring apparatus (34) for measuring the pressure in the fluid system (B) including the dialysate chamber (3) for carrying out an integrity test for monitoring the integrity of the dialyser (1),
   a control and arithmetic unit (29) which cooperates with the pump system (38) and the pressure measuring apparatus (34) and which provides a preparation mode in preparation for the blood treatment,
   **characterised in that**, for carrying out the preparation mode, the control and arithmetic unit (29) is so configured that the fluid system (B) including the dialysate chamber (3) is filled with a liquid, the integrity test for monitoring the integrity of the dialyser (2) is carried out with a semipermeable membrane (4) wetted only on the dialysate side after the fluid system (B) including the dialysate chamber (3) has been filled with a liquid, and the blood chamber (2) is filled with a liquid after the integrity test has been carried out, the control and arithmetic unit (29) being so configured for carrying out the integrity test in the preparation mode that a pressure difference is established between the blood chamber (2) and the dialysate chamber (3) in such a manner that the pressure in the blood chamber is greater than the pressure in the dialysate chamber, the increase in the pressure p, measured by the pressure measuring apparatus (34), in a given time interval being determined, and a leakage rate LR being determined from the increase in the pressure in a given time interval and the compliance C of the fluid system (B) including the dialysate chamber (3), and a lack of integrity of the dialyser being concluded on the basis of the determined leakage rate LR.

2. Extracorporeal blood treatment device according to claim 1, **characterised in that** the control and arithmetic unit (29) is so configured that, for establishing the pressure difference between the blood chamber (2) and the dialysate chamber (3), liquid is removed by means of the pump system (38) from the fluid system (B) including the dialysate chamber (3) or the pressure in the blood chamber (2) is increased by means of the pump system (38) by supplying air or liquid to the extracorporeal blood circuit (A).

3. Extracorporeal blood treatment device according to either claim 1 or claim 2, **characterised in that** the control and arithmetic unit (29) is so configured that the leakage rate LR is calculated according to the following equation:

$$LR = C \, dp/dt$$

   where C is the compliance C of the fluid system (B) including the dialysate chamber, and dp/dt is the rate of pressure increase.

4. Extracorporeal blood treatment device according to claim 1, 2 or 3, **characterised in that** the control and arithmetic unit (29) is so configured that, for monitoring the integrity of the dialyser (1), in the preparation mode the compliance C is calculated from a volume of the liquid which is removed from the fluid system (B) including the dialysate chamber (3) and the fall in the pressure measured by means of the pressure measuring apparatus (34).

5. Extracorporeal blood treatment device according to claim 4, **characterised in that** the control and arithmetic unit (29) is so configured that the compliance C is calculated according to the following equation:

$$C = dV/dp$$

   where dV is the volume of liquid removed from the fluid system (B) including the dialysate chamber, and dp is the fall in pressure before removal and after removal of the liquid.

6. Extracorporeal blood treatment device according to any of claims 1 to 5, **characterised in that** the control and arithmetic unit (29) is so configured that the determined leakage rate LR is compared with a given limit value for the leakage rate and a lack of integrity is concluded if the leakage rate does not fall below the given limit value in a given time interval.

7. Extracorporeal blood treatment device according to any of claims 2 to 6, **characterised in that** the pump system (38) has an ultrafiltrate pump (24), and **in that** the control and arithmetic unit (29) is so configured that the ultrafiltrate pump (24) is operated to remove liquid from the fluid system (B) including the dialysate chamber (3), such that a fall in pressure occurs in the fluid system (B) including the dialysate chamber (3).

8. Extracorporeal blood treatment device according to any of claims 1 to 7, **characterised in that** the control and arithmetic unit (29) is so configured that in the preparation mode, after the fluid system (B) including the dialysate chamber (3) has been filled with a liquid, the fluid system is flushed.

9. Method for monitoring the integrity of a dialyser of an extracorporeal blood treatment device for carrying out an extracorporeal blood treatment, in which blood flows in an extracorporeal blood circuit through the blood chamber of a dialyser which is separated by a semi-permeable membrane into the blood chamber and a dialysate chamber,

wherein the blood treatment device provides a preparation mode in preparation for the blood treatment, in which the fluid system including the dialysate chamber is filled with a liquid, an integrity test for monitoring the integrity of the dialyser is carried out with a semipermeable membrane wetted only on the dialysate side after the fluid system including the dialysate chamber has been filled, and the blood chamber is filled with a liquid after the integrity test has been carried out,

wherein the method for the integrity test in the preparation mode comprises the following method steps::

with a semipermeable membrane wetted only on the dialysate side establishing a pressure difference between the blood chamber and the dialysate chamber, such that the pressure in the blood chamber is greater than the pressure in the dialysate chamber,

determining the increase in the pressure p in the fluid system including the dialysate chamber in a given time interval, and determining a leakage rate LR from the increase in the pressure in a given time interval and the compliance C of the fluid system including the dialysate chamber, and

generating a control signal signalling the lack of integrity of the dialyser on the basis of the determined leakage rate.

10. Method for monitoring the integrity of a dialyser according to claim 9, **characterised in that**, for establishing a pressure difference between the blood chamber and the dialysate chamber, liquid is removed from the fluid system including the dialysate chamber or the pressure in the blood chamber (2) is increased by means of the pump system (38) by supplying air or liquid to the extracorporeal blood circuit (A).

11. Method for monitoring the integrity of a dialyser according to either claim 9 or claim 10, **characterised in that** the leakage rate LR is determined according to the following equation:

$$LR = C\, dp/dt$$

where C is the compliance C of the fluid system including the dialysate chamber, and dp/dt is the rate of pressure increase.

12. Method for monitoring the integrity of a dialyser according to claim 9, 10 or 11, **characterised in that**, for monitoring the integrity of the dialyser, in the preparation mode the compliance C is calculated from a volume of the liquid which is removed from the fluid system including the dialysate chamber, and the fall in the measured pressure p.

13. Method for monitoring the integrity of a dialyser according to claim 12, **characterised in that** the compliance C of the fluid system is calculated according to the following equation:

$$C = dV/dp$$

where dV is the volume of liquid removed from the fluid system including the dialysate chamber, and dp is the fall in pressure before removal and after removal of the liquid.

14. Method for monitoring the integrity of a dialyser according to any of claims 9 to 13, **characterised in that** the determined leakage rate LR is compared with a given limit value for the leakage rate, and a control signal signalling the lack of integrity of the dialyser is generated if the leakage rate does not fall below the given limit value in a given time interval.

15. Method for monitoring the integrity of a dialyser according to any of claims 9 to 14, **characterised in that**, after the fluid system including the dialysate chamber has been filled with a liquid, the fluid system is flushed using the liquid.

**Revendications**

1. Dispositif de traitement extracorporel du sang pour la mise en oeuvre d'un traitement extracorporel du sang, pour lequel du sang s'écoule dans une circulation sanguine extracorporelle (A) à travers la chambre à sang (2) d'un dialyseur (1), qui est divisé par une membrane semiperméable (4) en la chambre à sang (2) et une chambre à dialysat (3),

dans lequel le dispositif de traitement du sang présente :

un système fluidique (B) enfermant la chambre à dialysat (3) au moins lors du fonctionnement, un système de pompage (38) pour le remplissage avec un liquide du système fluidique (B) enfermant la chambre à dialysat (3) et pour l'établissement d'une différence de pression entre la chambre à sang (2) et la chambre à dialysat (3),

dans lequel le système de pompage (38) présente au moins une pompe (6, 17, 24),

un équipement de mesure de pression (34) pour la mesure de la pression dans le système fluidique (B) enfermant la chambre à dialysat (3) pour la mise en oeuvre d'un test d'intégrité pour la surveillance de l'intégrité du dialyseur (1),

une unité de commande et de calcul (29) coopérant avec le système de pompage (38) et l'équipement de mesure de pression (34), qui prévoit un mode de préparation préparant le traitement du sang,

**caractérisé en ce que**, pour la mise en oeuvre du mode de préparation, l'unité de commande et de calcul (29) est configurée de telle sorte que le système fluidique (B) enfermant la chambre à dialysat (3) est rempli d'un liquide, qu'après le remplissage du système fluidique (B) enfermant la chambre à dialysat (3) avec un liquide, le test d'intégrité pour la surveillance de l'intégrité du dialyseur (2) est mis en oeuvre avec une membrane semiperméable (4) humidifiée uniquement du côté du dialysat, et qu'après la mise en oeuvre du test d'intégrité la chambre à sang (2) est remplie d'un liquide, dans lequel

l'unité de commande et de calcul (29) pour la mise en oeuvre du test d'intégrité dans le mode de préparation est configurée de telle sorte qu'une différence de pression est établie entre la chambre à sang (2) et la chambre à dialysat (3), de telle sorte que la pression dans la chambre à sang est supérieure à la pression dans la chambre à dialysat,

l'augmentation de la pression p mesurée avec l'équipement de mesure de pression (34) dans un intervalle de temps prédéfini est déterminée, et

un taux de fuite LR est déterminé à partir de l'augmentation de la pression dans un intervalle de temps prédéfini et de la compliance C du système fluidique (B) enfermant la chambre à dialysat (3), et

un manque d'intégrité du dialyseur est déduit sur la base du taux de fuite LR déterminé.

2. Dispositif de traitement extracorporel du sang selon la revendication 1, **caractérisé en ce que** l'unité de commande et de calcul (29) est configurée de telle sorte que pour l'établissement de la différence de pression entre la chambre à sang (2) et la chambre à dialysat (3) avec le système de pompage (38), du liquide est retiré du système fluidique (B) enfermant la chambre à dialysat (3) ou la pression dans la chambre à sang (2) est augmentée par amenée d'air ou de liquide dans la circulation sanguine extracorporelle (A) avec le système de pompage (38).

3. Dispositif de traitement extracorporel du sang selon la revendication 1 ou 2, **caractérisé en ce que** l'unité de commande et de calcul (29) est configurée de telle sorte que le taux de fuite LR est calculé selon l'équation suivante :

$$LR = C \, dp/dt$$

où C est la compliance C du système fluidique (B) enfermant la chambre à dialysat et dp/dt est le taux d'augmentation de pression.

4. Dispositif de traitement extracorporel du sang selon la revendication 1, 2 ou 3, **caractérisé en ce que** l'unité de commande et de calcul (29) est configurée de telle sorte que pour la surveillance de l'intégrité du dialyseur (2) dans le mode de préparation la compliance C est calculée à partir d'un volume du liquide qui est retiré du système fluidique (B) enfermant la chambre à dialysat (3), et de la diminution de la pression mesurée avec l'équipement de mesure de pression (34).

5. Dispositif de traitement extracorporel du sang selon la revendication 4, **caractérisé en ce que** l'unité de commande et de calcul (29) est configurée de telle sorte que la compliance C est calculée selon l'équation suivante :

$$C = dV/dp$$

où dV est le volume de liquide retiré du système fluidique (B) enfermant la chambre à dialysat et dp la diminution de la pression avant le retrait et après le retrait du liquide.

6. Dispositif de traitement extracorporel du sang selon l'une quelconque des revendications 1 à 5, **caractérisé en ce que** l'unité de commande et de calcul (29) est configurée de telle sorte que le taux de fuite LR déterminé est comparé à une valeur limite prédéfinie pour le taux de fuite et un manque d'intégrité est déduit lorsque la valeur limite prédéfinie pour le taux de fuite n'est pas atteinte dans un intervalle de temps prédéfini.

**7.** Dispositif de traitement extracorporel du sang selon l'une quelconque des revendications 2 à 6, **caractérisé en ce que** le système de pompage (38) présente une pompe à ultrafiltrat (24), et que l'unité de commande et de calcul (29) est configurée de telle sorte que la pompe à ultrafiltrat (24) fonctionne pour retirer du liquide du système fluidique (B) enfermant la chambre à dialysat (3), de sorte qu'une diminution de pression s'effectue dans le système fluidique (B) enfermant la chambre à dialysat (3).

**8.** Dispositif de traitement extracorporel du sang selon l'une quelconque des revendications 1 à 7, **caractérisé en ce que** l'unité de commande et de calcul (29) est configurée de telle sorte que le système fluidique est rincé avec un liquide dans le mode de préparation après le remplissage du système fluidique (B) enfermant la chambre à dialysat (3).

**9.** Procédé pour la surveillance de l'intégrité d'un dialyseur d'un dispositif de traitement extracorporel du sang pour la mise en oeuvre d'un traitement extracorporel du sang, pour lequel du sang circule dans une circulation sanguine extracorporelle à travers la chambre à sang d'un dialyseur, qui est divisé par une membrane semiperméable en la chambre à sang et une chambre à dialysat, dans lequel le dispositif de traitement du sang prévoit un mode de préparation préparant le traitement du sang, au cours duquel le système fluidique enfermant la chambre à dialysat est rempli d'un liquide, après le remplissage du système fluidique enfermant la chambre à dialysat un test d'intégrité pour la surveillance de l'intégrité du dialyseur est mis en oeuvre avec une membrane semiperméable humidifiée uniquement du côté du dialysat, et après la mise en oeuvre du test d'intégrité la chambre à sang est remplie d'un liquide, dans lequel le procédé pour le test d'intégrité dans le mode de préparation comprend les étapes de procédé suivantes :

avec une membrane semiperméable humidifiée uniquement du côté du dialysat, l'établissement d'une différence de pression entre la chambre à sang et la chambre à dialysat, de telle sorte que la pression dans la chambre à sang dépasse la pression dans la chambre à dialysat, la détermination de l'augmentation de la pression p dans le système fluidique enfermant la chambre à dialysat dans un intervalle de temps prédéfini, et la détermination, à partir de l'augmentation de la pression dans un intervalle de temps prédéfini et de la compliance C du système fluidique enfermant la chambre à dialysat, d'un taux de fuite LR, et la génération d'un signal de commande signalant le manque d'intégrité du dialyseur sur la base du taux de fuite déterminé.

**10.** Procédé pour la surveillance de l'intégrité d'un dialyseur selon la revendication 9, **caractérisé en ce que**, pour l'établissement d'une différence de pression entre la chambre à sang et la chambre à dialysat, du liquide est retiré du système fluidique enfermant la chambre à dialysat ou la pression dans la chambre à sang (2) est augmentée par amenée d'air ou de liquide dans la circulation sanguine extracorporelle (A) avec le système de pompage (38).

**11.** Procédé pour la surveillance de l'intégrité d'un dialyseur selon la revendication 9 ou 10, **caractérisé en ce que** le taux de fuite LR est calculé selon l'équation suivante :

$$LR = C\ dp/dt$$

où C est la compliance C du système fluidique enfermant la chambre à dialysat et dp/dt le taux d'augmentation de pression.

**12.** Procédé pour la surveillance de l'intégrité d'un dialyseur selon la revendication 9, 10 ou 11, **caractérisé en ce que** pour la surveillance de l'intégrité du dialyseur dans le mode de préparation la compliance C est calculée à partir d'un volume du liquide qui est retiré du système fluidique enfermant la chambre à dialysat, et de la diminution de la pression p mesurée.

**13.** Procédé pour la surveillance de l'intégrité d'un dialyseur selon la revendication 12, **caractérisé en ce que** la compliance C du système fluidique est calculée selon l'équation suivante :

$$C = dV/dp$$

où dV est le volume de liquide retiré du système fluidique enfermant la chambre à dialysat et dp la diminution de la pression avant le retrait et après le retrait du liquide.

**14.** Procédé pour la surveillance de l'intégrité d'un dialyseur selon l'une quelconque des revendications 9 à 13, **caractérisé en ce que** le taux de fuite LR déterminé est comparé à une valeur limite prédéfinie pour le taux de fuite et un signal de commande signalant le manque d'intégrité du dialyseur est généré, lorsque la valeur limite prédéfinie pour le taux de fuite LR n'est pas atteinte dans un intervalle de temps prédéfini.

**15.** Procédé pour la surveillance de l'intégrité d'un dialyseur selon l'une quelconque des revendications 9 à 14, **caractérisé en ce que** le système fluidique est rincé avec un liquide après le remplissage du système fluidique enfermant la chambre à dialysat.

**Fig. 1**

Fig. 2

Fig. 3

**IN DER BESCHREIBUNG AUFGEFÜHRTE DOKUMENTE**

**In der Beschreibung aufgeführte Patentdokumente**

- WO 9711770 A1
- EP 1327457 A1